# EUROPEAN PATENT SPECIFICATION

(11) **EP 1 560 563 B1**
(45) Date of publication and mention of the grant of the patent: **01.07.2009**
(21) Application number: 03772608.0
(22) Date of filing: 04.11.2003
(51) Int. Cl.: A61K 9/00, A61N 1/30, A61M 31/00

(54) **IMPLANTABLE MEDICAL DEVICE FOR CONTROLLED RELEASE OF A SUBSTANCE**
MEDIZINISCHES IMPLANTAT ZUR KONTROLLIERTEN ABGABE EINER SUBSTANZ
DISPOSITIF MEDICAL IMPLANTABLE POUR LA LIBERATION CONTROLEE D'UNE SUBSTANCE

(30) Priority: 04.11.2002 IL 15262902
(43) Date of publication of application: 10.08.2005
(73) Proprietor: Innoventions (Israel) Ltd., 38900 Caesarea Industrial Park (IL)
(72) Inventor: YACHIA, Daniel, 46762 Herzliya (IL); HIRSZOWICZ, Eran, 52236 Ramat Gan (IL)
(74) Representative: Casey, Lindsay Joseph
(86) International application number: PCT/IL2003/000914
(87) International publication number: WO 2004/041242

(56) References cited:
- WO-A-01/35928
- WO-A-01/41736
- WO-A-02/30401
- US-A- 6 123 861
- US-A1- 2002 082 551
- US-A1- 2002 119 176
- US-A1- 2002 138 067

## Description

### FIELD OF THE INVENTION

The invention is in the field of implantable medical devices. More specifically, the invention relates to such devices controlled release of a substance in a body cavity such as a urinary bladder or digestive tract organ.

### BACKGROUND OF THE INVENTION

There are many instances when it is desirable to release a substance in a body cavity over a prolonged period of time.

US Patent No. 6,364,856 to Ding et al., for example, discloses medical devices comprising an expandable portion which is covered with a sponge coating for releasing a biologically active material. The sponge coating is made of a non-hydrogel polymer having a plurality of voids. The device can further include means for infusing or expelling the biologically active material or drug into the voids. The drug is delivered to the body lumen of a patient by expelling the drug and inflating or expanding the expandable portion of the catheter or device.

US Patent No. 6,187,038 to Sullivan et al., discloses a composite graft for a blood vessel comprising: an inner vessel made of a biologic collagenic material and an outer sleeve surrounding the inner vessel and defining an annular gap between the inner vessel and the sleeve. A polymeric fabric, and a bioactive compound in the annular gap carried on a time-release vesicle.

US Patent 6,187,768 to Harle discloses corpuscles for implantation into or at body tissue. Medicine is distributed in carriers formed of biologically inert material for release into body tissue after implantation. The surface-to-volume ratio of the carriers is more satisfactory than that of the carriers forming part of conventional corpuscles so that they are readily withdrawn from the body.

US Patent Nos. 6,293,923 and 6,398,718 to Yachia et al describe devices for insertion into a urinary bladder that may be adapted to release a substance in the bladder.

International Patent Specification No WO-A-01135928 discloses apparatus and methods for providing for the delivery of molecules to a site via a carrier fluid. The apparatus includes microchip devices which have reservoirs containing the molecules for release. The apparatus and methods provide for active or passive controlled release of the molecules. Preferred embodiments include systems for intravenous administration of drugs, wherein drug molecules are released from the microchip devices into a carrier fluid *ex vivo*, such as a saline solution, forming a drug/saline solution mixture which is then delivered to a patient intravenously.

While some prior art devices allow the rate at which a substance is released from the device to be determined, prior art devices do not allow the release of the substance to be started and stopped repeatedly over time as may be required in any particular application.

According to an aspect of the present invention, there is provided a medical device as specified in claim 1.

In its second aspect, the invention thus provides a system for treating a body cavity of an individual, the system comprising:
(a) a device according to the invention and
(b) an applicator for inserting the device into the body or for removing the device from the body cavity, the applicator fitted at an end thereof with a gripping device for releasably gripping the device;

In its third aspect, the invention thus provides a system for treating a body cavity of an individual, the system comprising:
(a) a device according to the invention;
(b) an applicator for inserting the device into the body or for removing the device from the body cavity, the applicator fitted at an end thereof with a gripping device for releasably gripping the device; and
(c) an inflating device for inflating the balloon.

A method for releasing one or more substances into a body cavity containing an electrolytic fluid of an individual may comprise the steps of:
(a) loading the one or more substances into the vesicles of a device according to the invention;
(b) inserting the device into the body cavity;
(c) expanding the balloon in the urinary bladder; and
(d) displacing the balloon within the urinary bladder to a desired location.

A method for releasing one or more substances into a body cavity containing an electrolytic fluid of an individual may comprise the steps of:
(a) loading the one or more substances into the vesicles of a device according to the invention;
(b) inserting the device into the body cavity; and
(c) expanding the balloon in the body cavity.

### BRIEF DESCRIPTION OF THE DRAWINGS:

In order to understand the invention and to see how it may be carried out in practice, a preferred embodiment will now be described, by way of non-limiting example only, with reference to the accompanying drawings, in which:
**Fig. 1** shows a device for releasing substances in a body cavity in accordance with one embodiment of the invention;
**Fig. 2** shows a device for releasing substances in a body cavity in accordance with another embodiment of the invention;
**Fig. 3** shows the embodiment of Fig. 2 mounted on an inflatable balloon;
**Fig.4** shows the balloon of Fig. 3 without the device;
**Fig. 5** shows a portion of a balloon having a duck-bill valve;
**Fig. 6** shows a portion of a balloon according to the invention having a ball valve;
**Fig. 7** shows a device-balloon combination in which the balloon filled after have been inserted into body cavity;
**Fig. 8** shows a device-balloon combination in which the balloon is filled before being inserted into the urinary bladder;
**Fig. 9** shows use of an applicator for inserting a device-balloon combination into the urinary bladder of a female individual;
**Fig. 10** shows use of an applicator for inserting a device-balloon combination into the urinary bladder of a male individual;
**Fig. 11** shows a retrieval device for retrieving a device-balloon combination;
**Fig. 12** shows use of a displacing member to position a device-balloon combination in a desired position in a body cavity; and

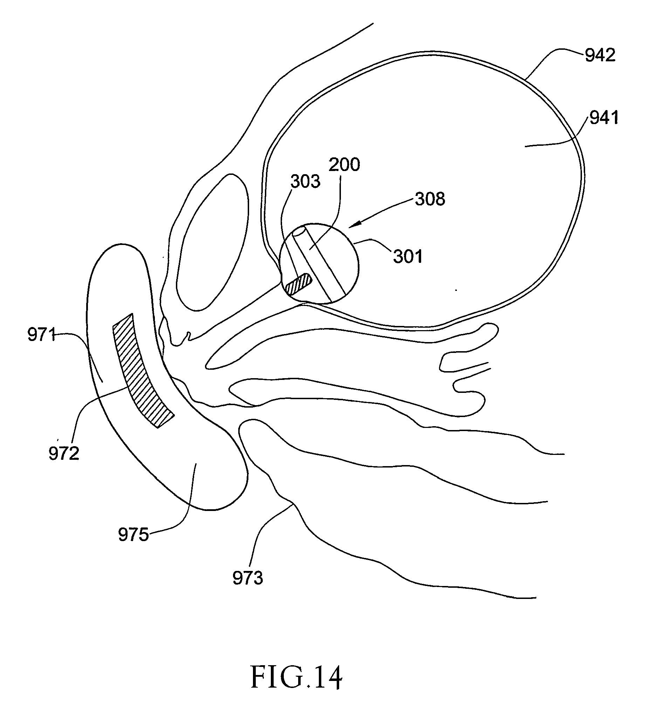

**Fig. 14** shows use of an immobilizing member.

### DETAILED DESCRIPTION OF SPECIFIC EMBODIMENTS

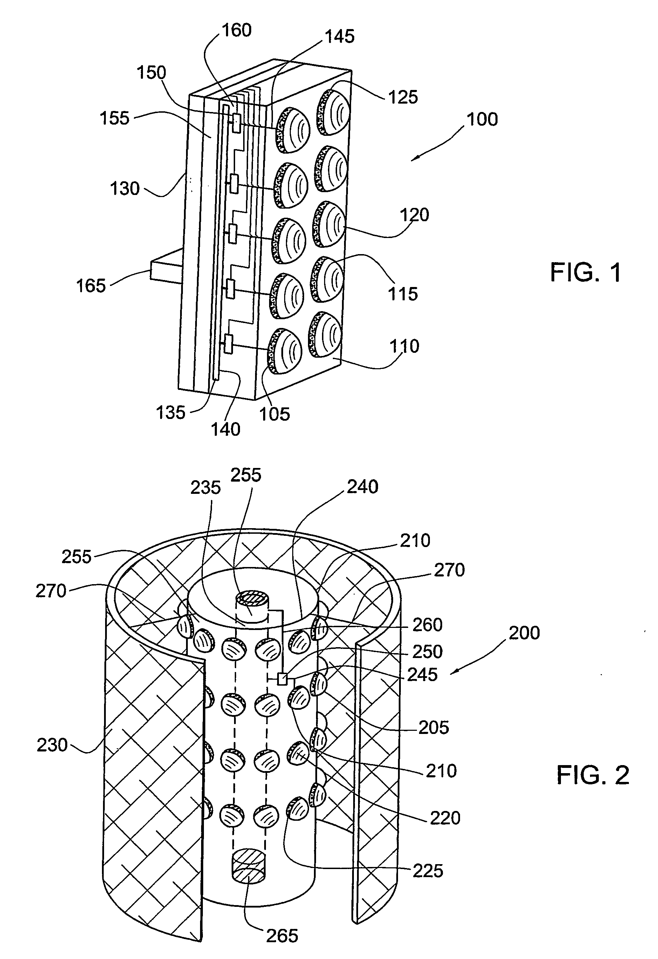

Reference is now made to Fig. 1 which shows a first embodiment 100 of a device for controlled released of a substance or substances into a body cavity in accordance with the invention. A plurality of blisters **105** are mounted on a first surface **110** of the device. The first surface **110** is formed from an insulating material such as rubber. Each blister **105** has a wall **115** surrounding a lumen 120. The lumen **120** of each blister is filled with the one or more substances (not shown) that are to be released into the body cavity. The wall **115** of each blister may be made entirely of metal, or may have a metallic portion. In the embodiment shown in Fig. 1, each blister has a metallic portion **125** that is referred to herein as the "*anode*". A second surface **130** of the device (referred to herein as the "*cathode*") **100** is formed from a second metal.

The device further comprises a power supply **135.** Each anode **125** is connected to a first terminal **140** of the power supply **135** via an individual electrical connection **145.** The connection **145** includes a switch **150.** The cathode **130** is connected to the second terminal of the power supply **135.**

The device may further include a processor **155** that is connected to each of the switches **150** by an individual connection **160** and is configured to close each switch at a predetermined time. For example, the processor **155** may be configured to close one switch every four hours, or some other predetermined time. Alternatively, the switches may be closed by means of a remote control located outside the body (not shown). For example, the user may be instructed to use the remote control to close a switch every evening before going to bed. As yet another example, the device 100 may include one or more detectors (not shown) for monitoring conditions within the body cavity that are input to the processor, the processor closing a switch under predetermined conditions inside the body cavity.

The device **100** may also include a magnet **165** in order to allow the device **100** to be positioned in the body cavity by means of a second magnet located outside the body (not shown).

Fig. 2 shows another embodiment **200** of the invention. In this embodiment a plurality of blisters **205** are mounted on a cylindrical surface **210.** The cylindrical surface **210** is formed from an insulating material such as rubber. Each blister **205** has a wall **215** surrounding a lumen **220.** The lumen **220** of each blister is filled with the one or more substances (not shown) that are to be released into the body cavity. The wall **215** of each blister may be made entirely of metal, or may have a metallic portion. In the embodiment shown in Fig. 2, each blister has a metallic portion **225** that is referred to herein as the "*anode* ".

In the interior of the cylindrical surface **210** is a power supply **235.** Each anode **225** is connected to a first terminal **240** of the power supply **235** via an individual electrical connection **245.** The connection **245** includes a switch **250.**

The interior of the cylindrical surface may further include a processor **255** that is connected to each of the switches **250** by an individual connection **260** and is configured to close each switch at a predetermined time. Alternatively, the switches may be closed by means of a remote control located outside the body (not shown).

Surrounding the cylindrical surface **240** is a cylindrically shaped cathode **230** that is coaxial with the cylindrical surface **240.** This arrangement is maintained by means of rigid radial rods **270.** The cathode **230** is formed from a second metal, and preferably has a mesh-like structure. The cathode **230** is connected to the second terminal of the power supply **135.** The

The device **200** may also include a magnet **265** in order to allow the device **200** to be positioned in the body cavity by means of a second magnet located outside the body (not shown).

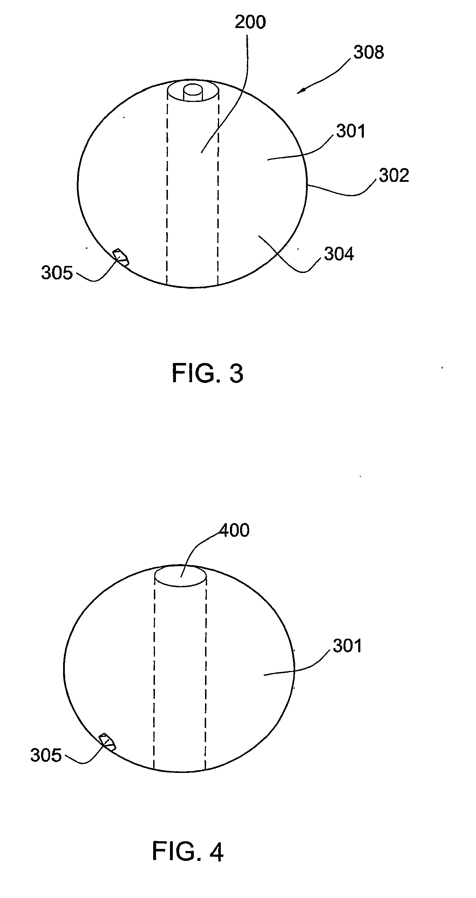

As shown in Fig. 3, the device **200** may be mounted on an inflatable balloon **301.** The balloon **302** made of a bio-compatible material enclosing a lumen **204**. The inflated balloon may have any desired shape as required in any particular application. In a preferred embodiment, the balloon **301** has a torroidal shape as shown in Fig. 3. The balloon **301** is shown in Fig. 4 without the device **200** being mounted in it. The balloon encircles a cylindrical hole **400** that is dimensioned to receive the device **200.** The device **200** is firmly set in the hole 400 by means of pressure exerted on the device **200** by the wall **202** of the balloon **301** when inflated.

The lumen **304** of balloon **301** may be filled with a bio-compatible fluid which may be pre-sterilized such as air, water, saline or an oil such as liquid paraffin. The balloon **301** may further comprise a magnetizable portions (not shown) in order to position the device in the body cavity by means of n external magnet. The magnetizable portion may consist for example, of one or more metal particles which may be free in the lumen **304,** attached to the inner surface of the wall **302or** embedded in the wall **302.**

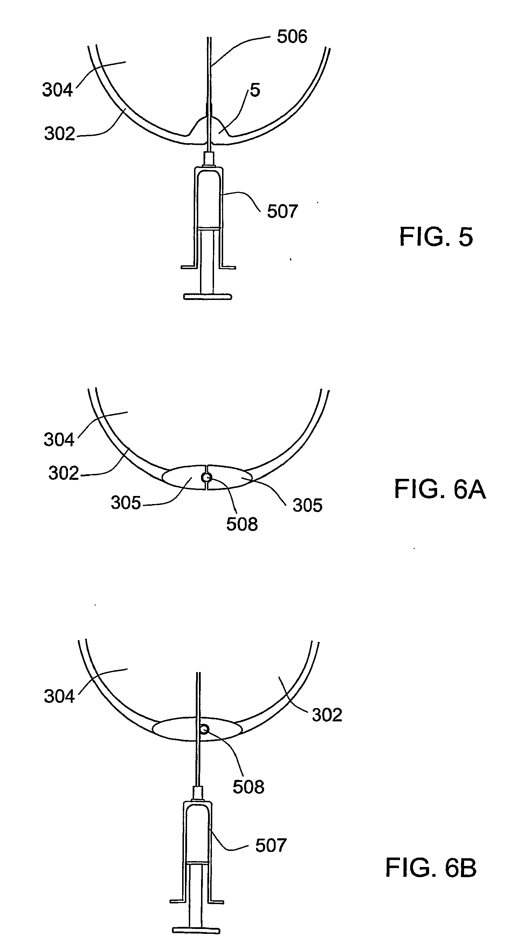

A self-sealing valve **305** in the wall of the balloon is used to fill the balloon. The valve 305 may be for example a duck-bill type valve as shown in Fig. 5, or a ball valve as shown in Fig. 6 in which a ball **508** may be in a sealing position (Fig. 6a) or an unsealing position (Fig. 6c). The canula **506** of a syringe **507** is inserted through the valve **305** into the lumen **304** of the balloon. Fluid injected into the lumen **304** causes the balloon **301** to expand. After filling, the syringe needle **506** is withdrawn, and the valve **305** seals itself. The inflated balloon with the device **200** mounted on it may float or sink in the electrolytic liquid in the body cavity..

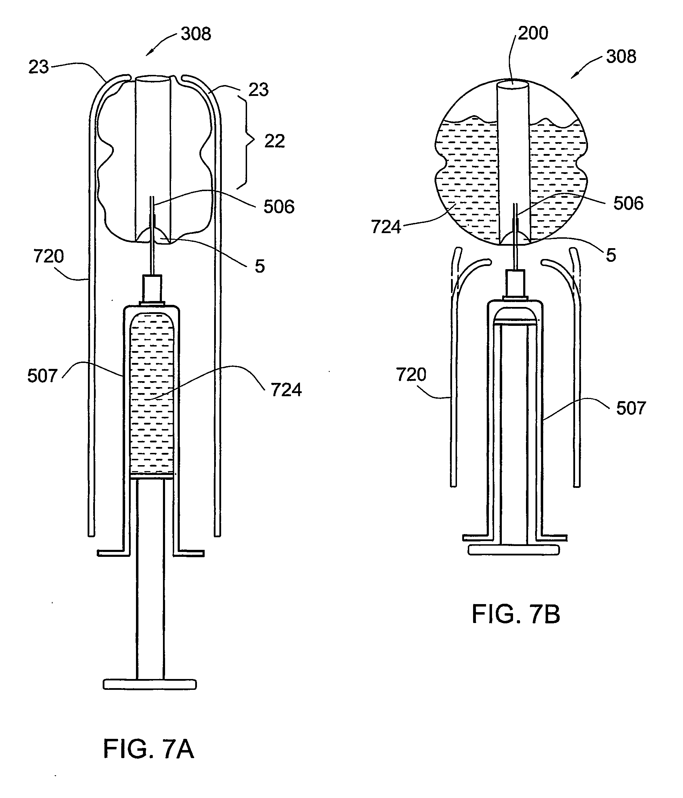

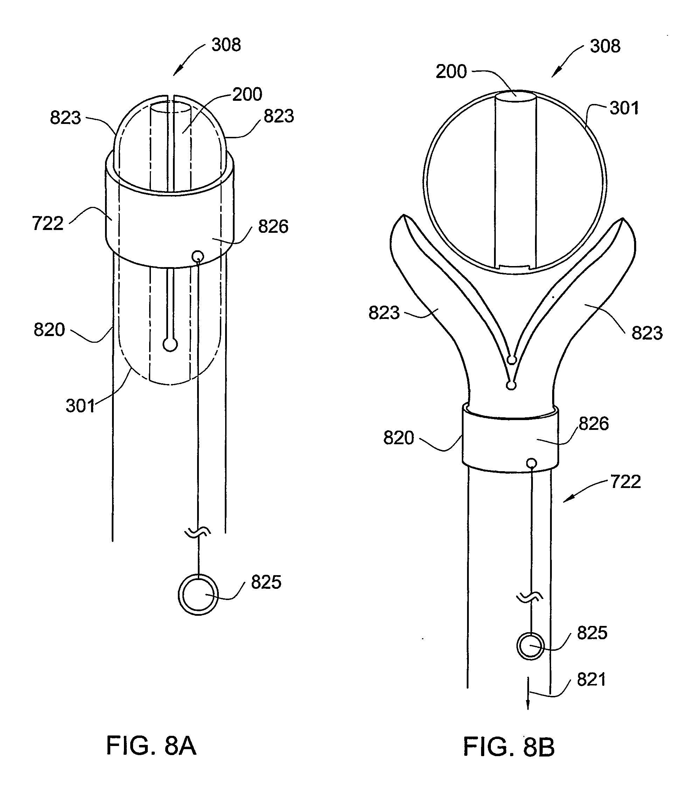

As shown in Fig. 7, the device-balloon combination **308** may first be delivered to the body cavity with the balloon **301** deflated, by means of an applicator **720** to be described below in detail (Fig. 7a). Following release of the device-balloon combination **308** from the applicator **720** into the cavity, the balloon **301** is filled with fluid **724** from the syringe **507** (Fig. 4b). Alternatively, as shown in Fig. 8a, the balloon **301** of the device-balloon combination **308** may be filled with a compressible fluid. The balloon **301** is then compressed before being inserted into the bladder by means of an applicator **820.** The device-balloon combination **308** with the pre-filled balloon is clutched by the flanges **823** which are initially kept closed by constraining sleeve **826** (Fig. 5a). After insertion of the applicator **820** with the device-balloon combination **308** into the body cavity, ring **825** is pulled as indicated by arrow **121** in Fig. 8b to urge the constraining sleeve **826** away from the flanges **823,** allowing flanges **823** to open and release the device-balloon combination **308** with the pre-filled balloon **301** into the body cavity.

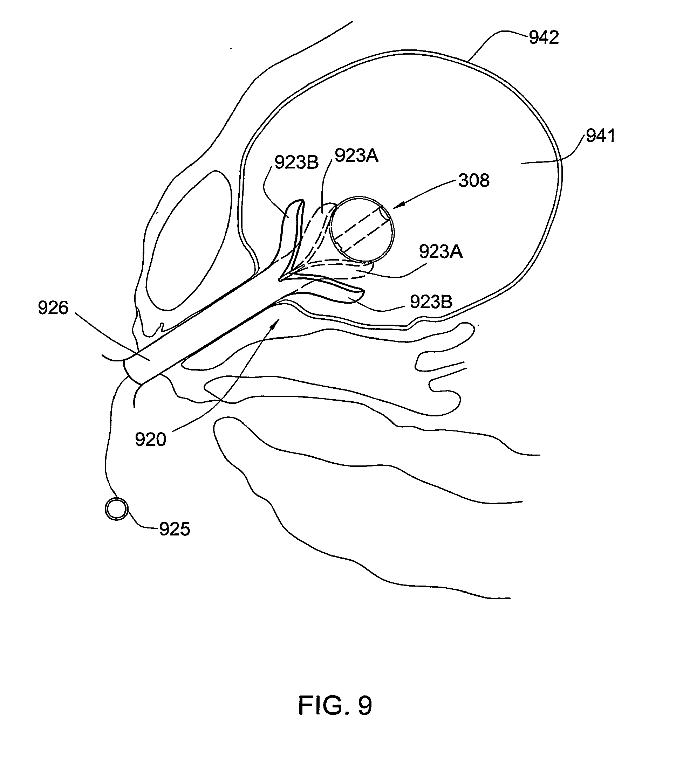

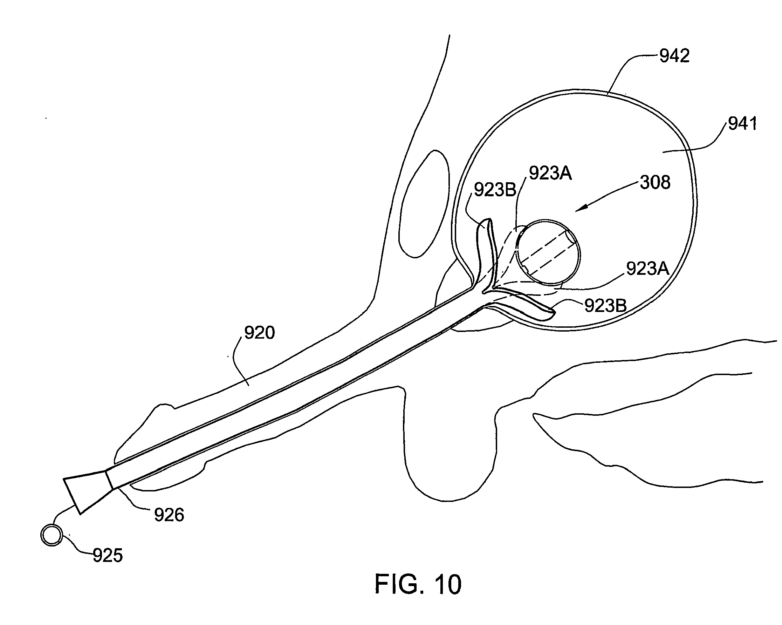

Fig. 9 shows use of an applicator **920** for inserting the device-balloon combination **308** into the lumen **941** of a urinary bladder **942** of a female individual, and Fig. 10 shows use of the applicator **920** inserting the device-balloon combination 308 into the lumen of the urinary bladder **942** of a male individual. In either case the device-balloon combination **308** is initially grasped by the closed flanges **923a** at the distal end of the applicator **920** (Figs. 9a and 10a). The distal end of the applicator with the device-balloon combination 308 is inserted into the urethra until it reaches the lumen **941** of the bladder **942.** The device-balloon combination 308 is then released from the applicator by opening the flanges **923b** by pulling on ring **925** while holding the constraining sleeve **926.** The applicator **920** is then removed from the body, leaving the device-balloon combination in the bladder lumen **41.**

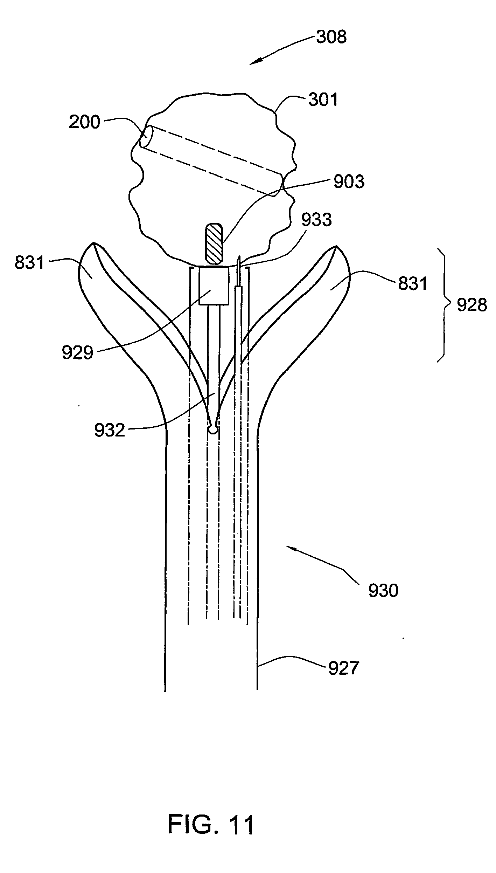

Fig. 11 shows a retrieval device generally designated as **930** for removing the device-balloon combination 308 from a body cavity. A catheter **927** has at its distal end **928** a magnetizable portion **929** so as to hold the device-balloon combination **308** at the distal tip **928** by means of the magneiztable particles associated with the balloon **301** or the device **200.**

The retrieval device **930** is inserted into the body cavity. After opening the flanges **831** of the retrieval device, the engaging probe **932** with magnetizable portion **929** in its tip is inserted into the body cavity so as to engage a magnet associated with either the device **200** or the balloon **301.** The probe **932** is then pulled so as to bring the balloon **301** into the grip of flanges **831** of the retrieval device **930.** A piercer **933** is inserted into the balloon **301** to drain the fluid **724** contained in its lumen **304** into an attached syringe (not shown) or into the body cavity. The retrieval device **930** is then withdrawn from the individual together with the device **200** and the deflated balloon **301.**

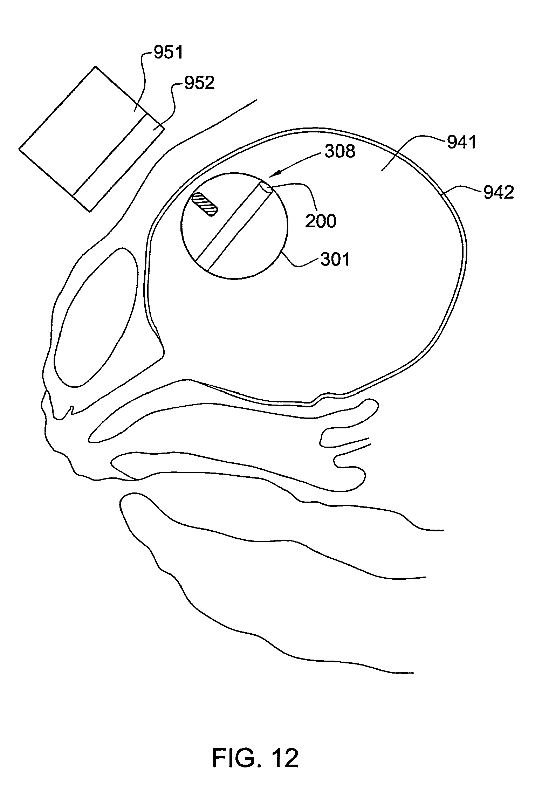

Fig. 12 shows use of a displacing member **951** to position the device-balloon combination 308 at a desired location in the body cavity. The displacing member **951** is located outside the individual's body and comprises a magnetizable portion **952.** The displacing member **951** is placed at a location on the surface of the individual's body so as to draw the device balloon combination to a desired location within the body cavity.

Fig. 13 shows use of an immobilizing member **971** comprising a magnetizable portion **972** affixed to the surface **973** of the individual's body so as to maintain the device-balloon combination 308 at the desired location in the body cavity. The magnetizable portion **972** of immobilizing member **971** may be enclosed in a coating **975** so as to form, for example, a hygienic pad. The immobilizing member **971** may be affixed to the surface **73** by means of tape, or may be incorporated into a garment worn by the individual.

The invention has been described with a certain degree of particularly only for the sake of clarity. However, several variations and modifications in the invention are possible within the scope of the following set of claims.

## Claims

1. A medical device (100, 200) for controlled release of one or more substances into a body cavity comprising:
(a) a power supply (135, 235) having first (140) and second terminals;
(b) a plurality of blister-like vesicles (105) mounted on a first surface (110) of the device (100, 200), each vesicle (105) having at least a metallic portion (125) formed from a first metal; a lumen of each blister being filled with one or more substances and,
(c) a cathode (130) formed from a second metal attached to the second terminal of the power supply (135);
(d) for each vesicle (105), an electrical connection (145) between the metallic portion (125) of the vesicle (105) and the first terminal (140) of the power supply (135, 235), each connection (145) including a switch (150) so as to allow the metallic portion (125) to function as an anode (125) when the switch (150) is closed; and
wherein the cathode (130) is separated from the anodes (125) by a space that is accessible by electrolytic fluid when the device (100, 200) is in the body cavity.

2. The device (100, 200) according to Claim 1 further comprising a processor (155) configured to close one or more switches (145) at one or more predetermined times.

3. The device (100, 200) according to Claim 1 further comprising one or more magnetizable particles.

4. The device (100, 200) according to Claim 1 wherein the switches (145) are closed by means of a remote control.

5. The device (100, 200) according to Claim 1, wherein the body cavity is a urinary bladder or a digestive tract organ.

6. The device (100, 200) according to Claim 1 wherein the anodes (125) are formed from copper and the cathode (130) is formed from zinc.

7. The device (100, 200) according to Claim 1 further comprising an inflatable balloon (301).

8. The device (100, 200) according to Claim 7, wherein the balloon (301) is formed with a magnetizable portion.

9. The device (100, 200) according to Claim 7 or 8 in which the balloon (301) further comprises a self-sealing valve (305).

10. The device (100, 200) according to any one of Claims 7 to 9, wherein the device (100, 200) after inflation of the balloon (301) floats in the electrolytic fluid.

11. The device (100, 200) according to any one of Claims 7 to 9, wherein the device (100, 200) after inflation of the balloon (100, 200) sinks in the electrolytic fluid.

12. The device (100, 200) according to any one of the previous claims wherein one or more of the one or more substances are drugs or antibiotics.

13. The device (100, 200) according to any one of the previous claims wherein one or more of the one or more substances are radioactive substances.

14. The device (100, 200) according to any one of the previous claims, further comprising one or more monitoring devices for parameters in the body cavity.

15. The device (100, 200) according to Claim 14, wherein one or more of the one or more of the monitoring devices is able to monitor a parameter of the body cavity selected from the list comprising:
(a) pressure of the electrolytic fluid;
(b) temperature of the electrolytic fluid;
(c) density of the electrolytic fluid ; and
(d) composition of the electrolytic fluid.

16. The device (100, 200) according to Claim 14 or 15 further comprising a processor (155) configured to receive data from a monitoring device and to close one or more switches (150) when under predetermined conditions in the body cavity.

17. A system for treating a body cavity of an individual, the system comprising:
(a) a device (100, 200) according to any one of the previous claims; and
(b) an applicator (720) for inserting the device (100, 200) into the body or for removing the device (100, 200) from the body cavity, the applicator (720) fitted at an end thereof with a gripping device for releasably gripping the device (100, 200);

18. A system for treating a body cavity of an individual, the system comprising:
(a) a device (100, 200) according to any one of Claims 7 to 16;
(b) an applicator (720) for inserting the device (100, 200) into the body or for removing the device (100, 200) from the body cavity, the applicator (720) fitted at an end thereof with a gripping device for releasably gripping the device; and
(c) an inflating device for inflating the balloon (301).

19. The system according to Claim 17 or 18 further comprising a magnetizable displacing member (951) for displacing the device (100, 200) within the body cavity.

20. The system according to any one of Claims 17 to 19, further comprising an immobilizing member (971) comprising a magnetizable portion (972), said immobilizing member (971) being secured onto the individual's body for immobilizing the device (100, 200) at a desired location in the body cavity.

21. The system according to Claim 20, wherein the immobilizing member (971) is in the form of a hygienic pad configured to be placed in a garment of the individual.

22. The system according to any one of Claims 17 to 21, wherein the gripping device comprises flanges (831).

23. The system according to any one of Claims 17 to 22, wherein the gripping device comprises a magnetizable portion.

24. The system according to Claim 18, wherein the inflating device comprises an injector for injecting a fluid into the balloon so as to expand the balloon (301).

## Patentansprüche

1. Medizinische Vorrichtung (100, 200) zur gesteuerten Abgabe einer oder mehrerer Substanzen in einen Körperhohlraum, die Folgendes umfasst:
(a) eine Energieversorgung (135, 235) mit einem ersten (140) und zweiten Anschluss;
(b) eine Mehrzahl blasenartiger Bläschen (105), die auf einer ersten Oberfläche (110) der Vorrichtung (100, 200) angebracht sind, wobei jedes Bläschen (105) mindestens einen aus einem ersten Metall gebildeten Metallteil (125) aufweist; und ein Lumen jeder Blase mit einer oder mehreren Substanzen gefüllt ist, und,
(c) eine Kathode (130), die aus einem zweiten Metall gebildet ist und an dem zweiten Anschluss der Energieversorgung (135) befestigt ist;
(d) für jedes Bläschen (105) eine elektrische Verbindung (145) zwischen dem Metallteil (125) des Bläschens (105) und dem ersten Anschluss (140) der Energieversorgung (135, 235), wobei jede Verbindung (145) einen Schalter (150) umfasst, damit der Metallteil (125) als eine Anode (125) wirken kann, wenn der Schalter (150) geschlossen ist; und
wobei die Kathode (130) von den Anoden (125) durch einen Raum getrennt ist, der für elektrolytische Flüssigkeit zugänglich ist, wenn sich die Vorrichtung (100, 200) in dem Körperhohlraum befindet.

2. Vorrichtung (100, 200) nach Anspruch 1, die weiter einen Prozessor (155) aufweist, der zum Schließen eines oder mehrerer Schalter (145) zu einer oder mehreren vorbestimmten Zeiten konfiguriert ist.

3. Vorrichtung (100, 200) nach Anspruch 1, die weiter eine oder mehrere magnetisierbare Partikeln aufweist.

4. Vorrichtung (100, 200) nach Anspruch 1, bei der die Schalter (145) mittels einer Fernsteuerung geschlossen werden.

5. Vorrichtung (100, 200) nach Anspruch 1, bei der der Körperhohlraum eine Harnblase oder ein Organ des Verdauungstraktes ist.

6. Vorrichtung (100, 200) nach Anspruch 1, bei der die Anoden (125) aus Kupfer gebildet sind und die Kathode (130) aus Zink gebildet ist.

7. Vorrichtung (100, 200) nach Anspruch 1, die weiter einen aufpumpbaren Ballon (301) aufweist.

8. Vorrichtung (100, 200) nach Anspruch 7, bei der der Ballon (301) mit einem magnetisierbaren Teil ausgebildet ist.

9. Vorrichtung (100, 200) nach Anspruch 7 oder 8, bei der der Ballon (301) weiter ein selbstschließende Ventil (305) aufweist.

10. Vorrichtung (100, 200) nach einem der Ansprüche 7 bis 9, wobei die Vorrichtung (100, 200) nach Aufpumpen des Ballons (301) in der elektrolytischen Flüssigkeit schwimmt.

11. Vorrichtung (100, 200) nach einem der Ansprüche 7 bis 9, wobei die Vorrichtung (100, 200) nach Aufpumpen des Ballons (100, 200) in die elektrolytische Flüssigkeit sinkt.

12. Vorrichtung (100, 200) nach einem der vorhergehenden Ansprüche, bei der eine oder mehrere der einen oder mehreren Substanzen Arzneimittel oder Antibiotika sind.

13. Vorrichtung (100, 200) nach einem der vorhergehenden Ansprüche, bei der eine oder mehrere der einen oder mehreren Substanzen radioaktive Substanzen sind.

14. Vorrichtung (100, 200) nach einem der vorhergehenden Ansprüche, die weiter eine oder mehrere Kontrolleinrichtungen für Parameter in dem Körperhohlraum aufweist.

15. Vorrichtung (100, 200) nach Anspruch 14, bei der eine oder mehrere der einen oder mehreren Kontrolleinrichtungen ein Parameter des Körperhohlraums kontrollieren kann/können, das aus der Folgendes umfassenden Liste ausgewählt ist:
(a) Druck der elektrolytischen Flüssigkeit;
(b) Temperatur der elektrolytischen Flüssigkeit;
(c) Dichte der elektrolytischen Flüssigkeit; und
(d) Zusammensetzung der elektrolytischen Flüssigkeit.

16. Vorrichtung (100, 200) nach Anspruch 14 oder 15, die weiter einen Prozessor (155) aufweist, der zum Empfangen von Daten von einer Kontrolleinrichtung und unter vorbestimmten Bedingungen in dem Körperhohlraum zum Schließen eines oder mehrerer Schalter (150) konfiguriert ist.

17. System zum Behandeln eines Körperhohlraums einer Person, wobei das System Folgendes umfasst:
(a) eine Vorrichtung (100, 200) nach einem der vorhergehenden Ansprüche; und
(b) einen Applikator (720) zum Einführen der Vorrichtung (100, 200) in den Körper oder zum Entfernen der Vorrichtung (100, 200) aus dem Körperhohlraum, wobei der Applikator (720) an einem Ende desselben mit einer Greifeinrichtung zum lösbaren Ergreifen der Vorrichtung (100, 200) ausgestattet ist;

18. System zum Behandeln eines Körperhohlraums einer Person, wobei das System Folgendes umfasst:
(a) eine Vorrichtung (100, 200) nach einem der Ansprüche 7 bis 16;
(b) einen Applikator (720) zum Einführen der Vorrichtung (100, 200) in den Körper oder zum Entfernen der Vorrichtung (100, 200) aus dem Körperhohlraum, wobei der Applikator (720) an einem Ende desselben mit einer Greifeinrichtung zum lösbaren Ergreifen der Vorrichtung ausgestattet ist; und
(c) eine Aufpumpeinrichtung zum Aufpumpen des Ballons (301).

19. System nach Anspruch 17 oder 18, das weiter ein magnetisierbares Verschiebeelement (951) zum Verschieben der Vorrichtung (100, 200) innerhalb des Körperhohlraums aufweist.

20. System nach einem der Ansprüche 17 bis 19, das weiter ein Feststellelement (971) mit einem magnetisierbaren Teil (972) aufweist, wobei das Feststellelement (971) an dem Körper der Person zum Feststellen der Vorrichtung (100, 200) an einer gewünschten Stellung in dem Körperhohlraum befestigt wird.

21. System nach Anspruch 20, bei dem das Feststellelement (971) in Form einer Hygienebinde vorliegt, die konfiguriert ist, um in ein Kleidungsstück der Person gelegt zu werden.

22. System nach einem der Ansprüche 17 bis 21, bei dem die Greifeinrichtung Flansche (831) aufweist.

23. System nach einem der Ansprüche 17 bis 22, bei dem die Greifeinrichtung einen magnetisierbaren Teil aufweist.

24. System nach Anspruch 18, bei dem die Aufpumpeinrichtung einen Injektor zum Injizieren eines Fluids in den Ballon aufweist, um so den Ballon (301) aufzupumpen.

## Revendications

1. Dispositif médical (100, 200) pour la libération contrôlée d'une ou de plusieurs substances dans une cavité corporelle comportant :
(a) une alimentation électrique (135, 235) ayant une première borne (140) et une deuxième borne ;
(b) une pluralité de cloques similaires à des alvéoles (105) montées sur une première surface (110) du dispositif (100, 200), chaque cloque (105) ayant au moins une partie métallique (125) formée à partir d'un premier métal ; une lumière de chaque alvéole étant remplie d'une ou de plusieurs substances et,
(c) une cathode (130) formée à partir d'un deuxième métal, attachée à la deuxième borne de l'alimentation électrique (135) ;
(d) pour chaque cloque (105), une connexion électrique (145) entre la partie métallique (125) de la cloque (105) et la première borne (140) de l'alimentation électrique (135, 235), chaque connexion (145) comprenant un commutateur (150) de manière à permettre à la partie métallique (125) de fonctionner en tant qu'anode (125) quand le commutateur (150) est fermé ; et
dans lequel la cathode (130) est séparée des anodes (125) par un espace auquel le fluide électrolytique a accès quand le dispositif (100, 200) est dans la cavité corporelle.

2. Dispositif (100, 200) selon la revendication 1, comportant par ailleurs un processeur (155) configuré pour fermer un ou plusieurs commutateurs (145) une fois ou un nombre prédéterminé de fois.

3. Dispositif (100, 200) selon la revendication 1, comportant par ailleurs une ou plusieurs particules magnétisables.

4. Dispositif (100, 200) selon la revendication 1, dans lequel les commutateurs (145) sont fermés au moyen d'une commande à distance.

5. Dispositif (100, 200) selon la revendication 1, dans lequel la cavité corporelle est une vessie urinaire ou un organe du tube digestif

6. Dispositif (100, 200) selon la revendication 1, dans lequel les anodes (125) sont formées à partir de cuivre et la cathode (130) est formée à partir de zinc.

7. Dispositif (100, 200) selon la revendication 1, comportant par ailleurs un ballonnet gonflable (301).

8. Dispositif (100, 200) selon la revendication 7, dans lequel le ballonnet (301) est formé avec une partie magnétisable.

9. Dispositif (100, 200) selon la revendication 7 ou la revendication 8, dans lequel le ballonnet (301) comporte par ailleurs une valve auto-obturatrice (305).

10. Dispositif (100, 200) selon l'une quelconque des revendications 7 à 9, dans lequel le dispositif (100, 200) après gonflage du ballonnet (301) flotte dans le fluide électrolytique.

11. Dispositif (100, 200) selon l'une quelconque des revendications 7 à 9, dans lequel le dispositif (100, 200) après gonflage du ballonnet (100, 200) coule dans le fluide électrolytique.

12. Dispositif (100, 200) selon l'une quelconque des revendications précédentes, dans lequel une ou plusieurs desdites une ou plusieurs substances sont des médicaments ou des antibiotiques.

13. Dispositif (100, 200) selon l'une quelconque des revendications précédentes, dans lequel une ou plusieurs desdites une ou plusieurs substances sont des substances radioactives.

14. Dispositif (100, 200) selon l'une quelconque des revendications précédentes, comportant par ailleurs un ou plusieurs dispositifs de surveillance pour des paramètres dans la cavité corporelle.

15. Dispositif (100, 200) selon la revendication 14, dans lequel un ou plusieurs desdits un ou plusieurs dispositifs de surveillance est en mesure de surveiller un paramètre de la cavité corporelle sélectionné parmi la liste comportant :
(a) la pression du fluide électrolytique ;
(b) la température du fluide électrolytique ;
(c) la densité du fluide électrolytique ; et
(d) la composition du fluide électrolytique.

16. Dispositif (100, 200) selon la revendication 14 ou la revendication 15, comportant par ailleurs un processeur (155) configuré pour recevoir des données en provenance d'un dispositif de surveillance et pour fermer un ou plusieurs commutateurs (150) en présence de conditions prédéterminées dans la cavité corporelle.

17. Système pour le traitement d'une cavité corporelle d'un individu, le système comportant :
(a) un dispositif (100, 200) selon l'une quelconque des revendications précédentes ; et
(b) un applicateur (720) destiné à insérer le dispositif (100, 200) dans le corps ou à retirer le dispositif (100, 200) de la cavité corporelle, l'applicateur (720) étant muni, à une extrémité de celui-ci, d'un dispositif d'accrochage destiné à accrocher le dispositif (100, 200) de manière libérable ;

18. Système pour le traitement d'une cavité corporelle d'un individu, le système comportant :
(a) un dispositif (100, 200) selon l'une quelconque des revendications 7 à 16 ;
(b) un applicateur (720) destiné à insérer le dispositif (100, 200) dans le corps ou à retirer le dispositif (100, 200) de la cavité corporelle, l'applicateur (720) étant muni, à une extrémité de celui-ci, d'un dispositif d'accrochage destiné à accrocher le dispositif de manière libérable ; et
(c) un dispositif de gonflage destiné à gonfler le ballonnet (301).

19. Système selon la revendication 17 ou la revendication 18, comportant par ailleurs un organe de déplacement magnétisable (951) destiné à déplacer le dispositif (100, 200) à l'intérieur de la cavité corporelle.

20. Système selon l'une quelconque des revendications 17 à 19, comportant par ailleurs un organe d'immobilisation (971) comportant une partie magnétisable (972), ledit organe d'immobilisation (971) étant assujetti sur le corps de l'individu afin d'immobiliser le dispositif (100, 200) au niveau d'un emplacement souhaité dans la cavité corporelle.

21. Système selon la revendication 20, dans lequel l'organe d'immobilisation (971) est sous la forme d'une serviette hygiénique configurée pour être placée dans un vêtement de l'individu.

22. Système selon l'une quelconque des revendications 17 à 21, dans lequel le dispositif d'accrochage comporte des brides (831).

23. Système selon l'une quelconque des revendications 17 à 22, dans lequel le dispositif d'accrochage comporte une partie magnétisable.

24. Système selon la revendication 18, dans lequel le dispositif de gonflage comporte un injecteur destiné à injecter un fluide dans le ballonnet de manière à dilater le ballonnet (301).
